# EUROPEAN PATENT APPLICATION

(11) **EP 2 431 477 A1**
(43) Date of publication of application: **21.03.2012**
(21) Application number: 10774948.3
(22) Date of filing: 13.05.2010
(51) Int. Cl.: C12P 17/16, A61K 31/4433, A61P 31/10, A61P 33/02, A61P 33/08, C07D 405/04, C12N 1/14, C12R 1/645

(54) **POLYKETIDE COMPOUND**

(30) Priority: 14.05.2009 JP 2009117559
(71) Applicant: Astellas Pharma Inc., Tokyo 103-8411 (JP)
(72) Inventor: OHSUMI Keisuke, Tokyo 103-8411 (JP); MASAKI Teruhisa, Tsukuba-shi Ibaraki 103-8411 (JP)
(74) Representative: HOFFMANN EITLE
(86) International application number: PCT/JP2010/058076
(87) International publication number: WO 2010/131691

(57) **Abstract**

[Problem]

Provided is a compound which is useful as an agent for treating trichophytosis, protozoan infection and/or Pneumocystis pneumonia.

[Means for Solution]

Capnodiaceae strain No. 339855 was collected and a polyketide compound was isolated from the cultural solution thereof. It was confirmed that the polyketide compound or a salt thereof has a potent anti-*Trichophyton* activity and is useful as a pharmaceutical, particularly as an agent for treating trichophytosis, thereby completing the present invention. A cyclic compound of the present invention or a salt thereof may be used as agent for preventing or treating trichophytosis, protozoan infection and/or Pneumocystis pneumonia (formerly: carinii pneumonia).

## Description

### Technical Field

The present invention relates to a pharmaceutical composition, and particularly, to a cyclic compound which is useful as an active ingredient of a pharmaceutical composition for treating trichophytosis, protozoan infection and/or Pneumocystis pneumonia.

### Background Art

Among trichophytosis, for example, onychomycosis is caused by an infection of the nails by *Trichophyton* spp.(microorganism causing athlete's foot). Tinea pedis, also called athlete's foot, is caused by invasion of *Trichophyton* spp, from the skin on the feet into the nails when left untreated, and the main symptoms thereof are cloudy appearance and thickening of the nails.
Terbinafine (the following formula A) having squalene epoxidase inhibitory action, Griseofulvin (the following formula B) interfering with microtubule function, and Itraconazole (the following formula C) having the cytochrome P450 inhibitory action are used as agents for treating onychomycosis.

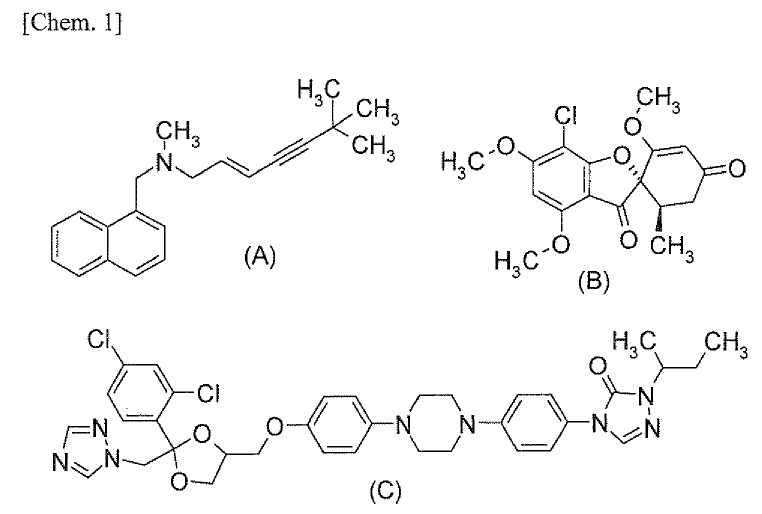

It is reported that Terbinafine has weak activity against dormantfungi present around infections (Mycoses, 46, pp. 505-510, 2003). In order to treat onychomycosis, the long-term administration of the drug is considered essential. Fungi which are growing, that is, fungi whose hyphae are growing, and fungi which are dormant, that is, fungi whose spores or hyphae are not growing, are present together in the nails having onychomycosis. Because growing fungi are terminated in the nails through which Terbinafine has permeated, the nails, which have been newly growing, are not infected by *Trichophyton* spp.. Since dormant fungi are not eradicated by Terbinafine, it is considered necessary to administer the drug for about 6 months until the nails grow again in order to provide fundamental treatment of onychomycosis. Therefore, even dormant fungi may be eradicated by a drug capable of killing the fungi before the nails grow again, and the treatment period may be shorten, and a high cure rate is expected.

Compounds having a 4-hydroxypyridine-2-one backbone may be, for example, Funiculosin (the following formula D, wherein --- represents a double bond), Tetrahydrofuniculosin (the following formula D, wherein --- represents a single bond), Ilicicolin H (the following formula E), etc. 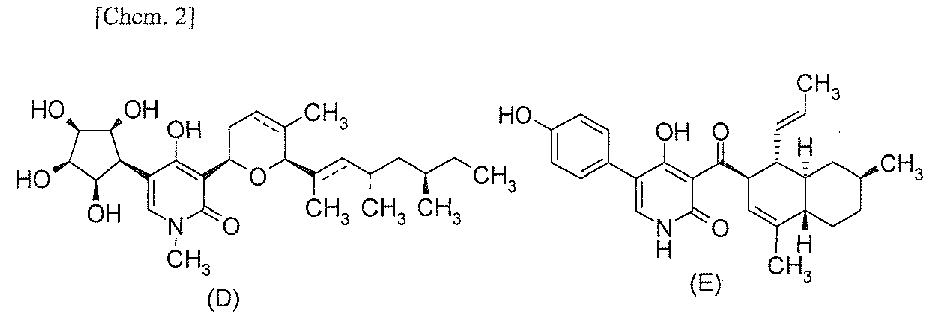 Funiculosin is an antifungal active material isolated from *Pehicillium funiculosum* cultural solution (Non-Patent Documents 1, 2, etc.), and Tetrahydrofuniculosin is a semi-synthetic compound made from Funiculosin (Patent Document 1 and Non-Patent Documents 3 and 4). Further, Ilicicolin H is an antifungal active material isolated from the imperfect fungus *Cylindrocladium iliciola* MFC-870 cultural solution (Non-Patent Documents 5 and 6).

### Prior Art

### Patent Document

Patent Document 1: Japanese Patent Application Laid-Open No. 53-121767

### Non-Patent Document

Non-Patent Document 1: Journal of Antibiotics, 31, pp.533-538, 1978
Non-Patent Document 2: Tetrahedron Letters, 41, pp.9397-9402, 2000
Non-Patent Document 3: Acta Crystallographica, Section C: Crystal Structure Communications, C46(3), pp.515-517, 1990
Non-Patent Document 4: Journal of Antibiotics, 31(6), pp.533-538, 1978
Non-Patent Document 5: Tetrahedron Letters, 17, pp.3827-3830, 1976
Non-Patent Document 6: Tetrahedron Letters, 46, pp.8009-8010, 2005

### Summary of Invention

### Problems to Be Solved by the Invention

It is an object of the present invention to provide a compound which is useful as an active ingredient of a pharmaceutical composition, and particularly, a pharmaceutical composition for treating trichophytosis, protozoan infection and/or Pneumocystis pneumonia.

### Means for Solving the Problems

The present inventors have earnestly studied a compound capable of treating trichophytosis, protozoan infection and/or Pneumocystis pneumonia, produced from microorganisms naturally present, and discovered the presence of a material which has excellent antimicrobial action against *Trichophyton* spp. and inhibit complex III in the cultural solution of Capnodiaceae strain No. 339855 which is a novel fungus discovered by the present inventors. The present inventors have further studied the subject, and succeeded in isolating a polyketide compound having excellent antifungal action from the cultural solution of the strain, thereby completing the present invention.

That is, the present invention relates to a compound of the formula (I) or a salt thereof, and a pharmaceutical composition including a compound of the formula (I) or a salt thereof and an excipient, and particularly to a pharmaceutical composition which is an agent for preventing or treating trichophytosis, protozoan infection and/or Pneumocystis pneumonia.

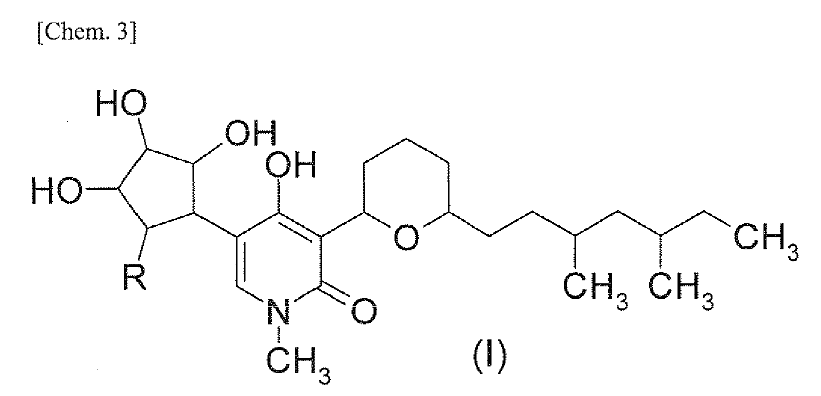

In another aspect, the present invention relates to a compound of the formula (I) or a salt thereof, which is for preventing or treating trichophytosis, protozoan infection and/or Pneumocystis pneumonia. In still another aspect, the present invention relates to a method for treating trichophytosis, protozoan infection and/or Pneumocystis pneumonia, including administering an effective amount of a compound of the formula (I) or a salt thereof to a patient,

The present invention relates to a method for preparing a compound of the formula (I) or a salt thereof, including culturing a microorganism that belongs to the family Capnodiaceae and has an activity of producing a compound of the formula (I), preferably, Capnodiaceae strain No. 339855 or variants thereof to isolate a compound of the formula (I) or a salt thereof from a culture thereof.

The present invention relates to Capnodiaceae strain No. 339855 which is a novel microorganism having an activity of producing a compound of the formula (I), which was deposited to the National Institute of Technology and Evaluation National Patent Microorganisms Depository Center as Accession No, NITE BP-735, or variants thereof.

### Effects of the Invention

A compound of the formula (I) or a salt thereof may be used as an agent for preventing and/or treating trichophytosis (for example, trichophytic granuloma, trichophytosis unguium, tinea manuum and pedis, trichophytosis corporis, trichophytosis cruris, tinea corporis and tinea cruris, trichophytosis capitis, Celsus kerion, sycosis trichophytica, Trichophytia profunda acuta of the glabrous skin and/or acute deep-seated trichopytosis the terminal hair, etc.), protozoan infection (for example, malaria, toxoplasmosis, etc.) and/or Pneumocystis pneumonia (formerly: carinii pneumonia), etc.

### Brief Description of Drawings

FIG. 1 is a view illustrating the ¹H-NMR spectrum of compound A (Solvent for measurement: CDCl₃).
FIG. 2 is a view illustrating the ¹³C-NMR spectrum of compound A (Solvent for measurement: CDCl₃).
FIG. 3 is a view illustrating the ¹H-NMR spectrum of compound B (Solvent for measurement: CDCl₃).
FIG. 4 is a view illustrating the ¹³C-NMR spectrum of compound B (Solvent for measurement: CDCl₃).

### Embodiments for Carrying Out the Invention

Hereinafter, the present invention will be described in detail.
The compound of the present invention may form a salt with a base. Examples of the salt may include inorganic bases, such as sodium, potassium, magnesium, calcium, etc. Further, the salt in the present invention also includes a so-called complex salt or a chelate compound.

A compound of the formula (I) may have 7 or 8 asymmetric carbon atoms, and optical isomers may be present based thereon. The present invention includes either a compound of the formula (I) isolated as a single optical isomer, or a compound of the formula (I) obtained as a mixture of a plurality of isomers.

The present invention also includes a pharmaceutically acceptable prodrug of the compound of the formula (I). The pharmaceutically acceptable prodrug is, for example, a prodrug chemically modified with a group well known to those skilled in the art, in which one or more -OH groups of the compound of the formula (I) can be converted into the original -OH by solvolysis or under physiological conditions.

The present invention also includes the compound of the formula (I), various hydrates or solvates of a salt thereof, and crystal polymorphic materials. Further, the present invention includes various compounds labeled with a radioisotope or a non-radioisotope.

Subsequently, mycological characteristics of a microorganism which produces the compound of the formula (I) will be described below.

### (1) Origin of Producing Strain

Capnodiaceae strain No. 339855, a type of fungus, was isolated by using a moist chamber method from fallen leave of a Japanese Zelkova, which was collected at Youroukeikoku in Otaki-Machi, Isumi-gun, Chiba, Japan. The strain was domestically deposited in the National Institute of Technology and Evaluation National Patent Microorganisms Depository Center (2-5-8 Kazusa-kamatari, Kisarazu, Chiba 292-0818, Japan), and given Accession No. NITE P-735 (Accession Date: April 16, 2009). Further, a request to transfer the strain to an international deposition organization based on the Budapest Treaty on January 29, 2010 (Transfer Date) was received, and the strain was given Accession No. BP-735 (Accession Date: April 16, 2009).

### (2) Morphological Characteristics of Producing Strain

The growth of the strain on a potato dextrose agar (Difco 2010) was good, and colonies grew to a diameter of 23 to 26 mm in 2 weeks at 25°C. The surfaces of the colonies were felty, and partially floccose. The peripheral portion of the colonies was in a circular pattern (entire), and a plurality of radial sulcates were formed from the center to the margin. The felty parts of the colonies were grayish green (30F8), while the floccose parts were dull grayish green (29E4). The other side of each colony was overall dark grayish green (28F4). Colonies attained 6 to 7 mm in diameter at 30°C two weeks later. No growth was observed at 5 and 37°C. The color descriptions were based on Methuen Handbook of Colour (Komerup, A. and J.H. Wanscher, 3rd ed., pp. 252, Methuen, London, 1987).

The growth of the strain on a corn meal agar (Difco 0386) was also good, and the colonies spread to a diameter of 22 to 24 mm in 2 weeks at 25°C. The surfaces of the colonies were velvety. The margins of the colonies were in a circular pattern (entire), and sulcates were not formed on the colonies. The colonies were grayish green (29F8-29F4). The colonies were not undulated, but smooth. The formation of drop-like conidial mass was observed at apex of conidiophore on the colonies. The other side of each colony was overall dark grayish green (28F4). Colonies attained 6 to 9 mm in diameter at 30°C two weeks later. No growth was observed at 5 and 37°C. The color descriptions were based on Methuen Handbook of Colour (Komerup, A. and J.H. Wanscher, 3rd ed., pp. 252, Methuen, London, 1987).

The strain was grown at 12 to 30°C, and the optimum growth temperature was in the range of 20 to 25°C. Data on the growth temperatures were measured on potato dextrose agar (Difco 2010). The pH at which the strain was capable of growing was in the range of 2.02 to 11.46, and the optimum growth pH was in the range of 5.45 to 6.15. The pH at which the strain was capable of growing was measured in YM Broth (Difco).

The morphological characteristics of the strain were determined on the basis of the culture of the strain on a corn meal agar. Vegetative hyphae were brown and 2.3 to 4.0 µm thick. Chlamydospores were not observed. Conidiophores were not branched, but formed determinate synnemata. The overall length was 200 to 260 µm, and the thickness was 8 to 10 µm. The base of the synnemata formed stroma to be widened, and the width was 30 to 40 µm. Although the structure of synnemata of this fungus was morphologically similar to the genus *Graphium,* the synnemata was a little swollen at the upper part and again narrowed at the apex. Further, several strands of setae, 20 µm long, were formed to surround the apex part. Colorless conidia were formed in the narrowed apex part, and become a drop. The conidia were elliptical to cylindrical, and the size was (3.2 to 3.6)x(1.7 to 1.76) µm. The surface appeared smooth by the observation with an optical microscope (x400).

The strain formed synnemata, and the apex thereof (upper part) was swollen. Further, several strands of setae were formed at the swollen apex, which was one of the characteristics of the strain. The strain is morphologically similar to the genus *Graphium,* However, 28SrDNA homology search result shows that this strain close to the genus *Capnodium,* and different from the genus *Graphium.* As of now, this strain was identified as a strain of Capnodiaceae, and designated as Capnodiaceae strain No. 339855.

### (3) Cultural Characteristics

The cultural characteristics of the strain were determined on commercially available media and media prepared in accordance with compositions described in literature. As a potato dextrose agar, a Sabouraud dextrose agar, an Emerson YpSs agar, a corn meal agar, and an oatmeal agar, Difco 2010, Difco 0109, Difco 0739, Difco 0386 and Difco 0552 were purchased, respectively. A malt extract agar, a Czapek solution agar and an MY20 agar medium were prepared in accordance with the compositions described in a JCM catalog (Nakase, T. 6th ed., pp. 617, Japan Collection of Microorganisms, the Institute of Physical and Chemical Research, Saitama, 1995).

Almost similar properties were shown on the potato dextrose agar, the Sabouraud dextrose agar, the oatmeal agar, the malt extract agar, the Czapek solution agar and the MY20 agar medium, and thus characteristics on the potato dextrose agar were representatively described in (2) above. Further, the formation of spores was confirmed only on the corn meal agar, and thus cultural characteristics on the corn meal agar were described as a characteristic medium in (2) above.

The strain sometimes shows artificial or naturally-occurring variations. The strain Capnodiaceae strain No. 339855 used in the present invention includes not only the naturally isolated microorganism, but also artificial variants caused by ultraviolet rays, radiation rays, chemical agents and the like, and naturally-occurring variants thereof.

### (Process of Production)

The compound of the present invention may be obtained by culturing a microorganism that belongs to the family Capnodiaceae and has an activity of producing the compound of the present invention, preferably, Capnodiaceae strain No. 339855 or variants thereof to isolate the compound of the present invention from a culture thereof. The microorganism may be cultured in accordance with general cultivation methods of microorganisms.

The medium to be used is not particularly limited, so long as the medium contains nutrient sources capable of being utilized by the fungus Capnodiaceae strain No. 339855. A synthetic medium, a semi-synthetic medium or a natural medium may be used. With regard to the composition of the medium, L-arabinose, D-xylose, D-glucose, D-fructose, sucrose, inositol, L-rhamnose, raffinose, D-mannitol, mannose, melibiose, lactose, D-galactose, maltose, trehalose, salicin, xanthine, chitin, starch, glucose, dextrin, glycerol, vegetable oil or the like may be used as the carbon source. As the nitrogen source, meat extract, peptone, gluten meal, cottonseed meal, soybean powder, peanut powder, fish meal, corn steep liquor, dry yeast, yeast extract, ammonium chloride, ammonium sulfate, ammonium nitrate, uric acid, or other organic or inorganic nitrogen sources may be used. If desired, sulfate, nitrate, carbonate, phosphate or the like of sodium, potassium, magnesium, calcium, zinc, iron, cobalt or the like may added as metal salts. Further, a compound for promoting generation or an antifoaming agent, such as methionine, cysteine, cystine, thiosulfate, methyl oleate, lard oil, silicon oil, surfactants or the like, may be added, if desired.

With respect to the culturing condition, it is generally preferred to culture the strain under aerobic conditions, in the temperature range of 12 to 30°C, preferably around 20 to 25°C. The culturing period may be appropriately selected in accordance with the composition of the medium or conditions of temperature, but is generally about 1 to 30 days, preferably about 2 to 7 days.

The compound of the present invention may be purified and isolated from a culture by methods for purifying and isolating a physiologically active substance from a culture of a typical microorganism. That is, a culture was subjected to extraction with an appropriate organic solvent, and an effective material is purified and isolated from the resulting extract. That is, the separation and purification is carried out, using an antifungal activity as an index, by means which utilize the solubility and the difference in solubility to an appropriate solvent or the like, and are used in preparing a typical physiologically active substance. These methods may be used alone, or repeatedly applied in combination thereof in any order, as necessary. As other methods for purification, a culture as it is, or after removing the fungus from the culture by centrifugation or filtration, may be subjected to methods which utilize the solubility and the difference in solubility to an appropriate solvent, the difference in the rate of precipitation from a solution, the difference in adsorptive affinity to various adsorbents, the difference in distribution between two liquid phases, or the like. For example, a culture liquid may be brought into contact with an appropriate carrier, and an adsorbed compound may be eluted with an appropriate solvent from the carrier to purify the compound. These methods may be used alone, or repeatedly applied in combination thereof in any order, as necessary.

The compound of the formula (I) of the present invention or a salt thereof may be prepared by culturing the fungus which produces the substance in a nutrient medium and using a typical method from a culture in which the compound has been accumulated. The microorganism used in a method for preparing the compound is not particularly limited, so long as the microorganism belongs to the family Capnodiaceae and has an activity of producing the compound. Capnodiaceae strain No. 339855 internationally deposited as Accession No. NITE BP-735 may be appropriately used.

A pharmaceutical composition including one, or two or more of the compounds of the formula (I) or a salt thereof as an active ingredient may be prepared by conventional methods, using excipients typically used in the field, such as pharmaceutical excipients, pharmaceutical carriers or the like.

Examples of administration include oral administration by tablets, pills, capsules, granules, powders, liquids and the like, and parenteral administration by injections (e.g., intraarticular, intravenous, intramuscular or the like), suppositories, ophthalmic solutions, ophthalmic ointments, transdermal liquids, ointments, transdermal attachments, transmucosal liquids, transmucosal plasters, inhalation agents and the like.

For a solid formulation for oral administration, tablets, powders, granules or the like may be used. Such a solid formulation may be prepared by mixing one or two or more of the active ingredients with at least one inert excipient, such as lactose, mannitol, glucose, hydroxypropyl cellulose, microcrystalline cellulose, starch, polyvinylpyrrolidone, and/or magnesium aluminate metasilicate and the like. The composition may contain inert additives, for example, lubricants such as magnesium stearate, disintegrators such as sodium carboxymethyl starch or the like, stabilizers, or auxiliary dissolution agents, in accordance with conventional methods. The tablets or pills may be coated with a sugar coating or a film of a gastric or enteric substance, if desired.

The liquid composition for oral administration includes pharmaceutically acceptable emulsions, solutions, suspensions, syrups, elixirs or the like, and contains conventional inert diluents, such as distilled water or ethanol. In addition to the inert diluents, the liquid composition may contain adjuvants (such as solubilizers, humectants, or suspending agents), sweeteners, flavors, aromatic agents or preservatives.

The injections for parenteral administration include sterile, aqueous or non-aqueous solutions, suspensions, and emulsions. The aqueous solvent includes, for example, distilled water for injections and physiological saline. The non-aqueous solvent includes, for example, propylene glycol, polyethylene glycol, vegetable oils such as olive oil, alcohols such as ethanol, polysorbate 80 (name in Japanese Pharmacopoeia), and the like. Such compositions may further contain isotonic agents, preservatives, humectants, emulsifying agents, dispersants, stabilizers, or dissolution aids. These compositions may be sterilized, for example, by filtration through a fungus retaining filter, blending of a germicide, or irradiation. Alternatively, the compositions may be used by first making them into sterile solid compositions and dissolving or suspending them in sterile water or other sterile solvents for injection use prior to their use.

External preparations include ointments, plasters, creams, jellies, poultices, sprays, lotions, ophthalmic solutions, ophthalmic ointments and the like. Such preparations contain typically-used ointment bases, lotion bases, aqueous or nonaqueous solutions, suspensions, emulsions or the like. The examples of the ointment or lotion bases are polyethylene glycol, propylene glycol, white petrolatum, white beeswax, polyoxyethylene hydrogenated castor oil, glyceryl monostearate, stearyl alcohol, cetyl alcohol, lauromacrogol, sorbitan sesquioleate and the like.

Transmucosal agents such as inhalation agents and transnasal agents may be used in solid, liquid or semi-solid forms, and may be prepared by conventional methods. For example, known excipients, pH adjusters, preservatives, surfactants, lubricants, stabilizers, thickeners or the like may be added, if desired. For the administration, appropriate devices for inhalation or insufflation may be used. For example, using known devices (such as an inhalation device for measured administration) or sprayers, the compound may be administered alone or in a powder form of a formulated mixture, or as a solution or suspension in combination with a pharmaceutically acceptable carrier. An inhalation device for dry powder or the like may be a device for single administration or plural administrations, and a dry powder or a powder-containing capsule may be used. Alternatively, the compound may be administered in the form of an aerosol spray under pressure, or the like, using an appropriate agent for ejection, for example, an appropriate gas such as chlorofluoroalkane, hydrofluoroalkane, carbon dioxide or the like.

In the case of oral administration, the usual dosage is about 0.01 to 100 mg/kg, preferably 0.1 to 10 mg/kg per day, which is administered in one portion or two to four portions. In the case of intravenous administration, the usual dosage is about 0.01 to 100 mg/kg per day, which is administered once or several times a day. The dose is approximately determined by considering, for example, symptoms, age, sex or the like of individual patient to be administered.

The compound of the formula (I) may be used together with various agents of treating or preventing diseases for which the compound of the formula (I) is considered to be effective. The administration may be carried out simultaneously or successively without interval or with an appropriate interval. Simultaneous administration may be performed in the form of a single formulation, or in the form of discrete formulations.

### Example

A method for preparing the compound of the formula (I) will be described in more detail with reference to the following Examples. However, the present invention is not limited to the compounds described in the following Examples. Further, the method for preparing the compound of the formula (I) is not limited to the specific methods described in the following Examples, and the compound of the formula (I) or may be prepared by the combination of these methods, or a conventional method obvious to those skilled in the art.

The following abbreviations may be used herein.
MeCN=Acetanitrile, MeOH=Methanol, MgSO₄·7H₂O=Magnesium sulfate heptahydrate, t-BuOH=tert-Butyl alcohol, TFA=Trifluoroacetic acid, HR ESI MS=High Resolution Electrospray Ionization Mass Spectrometry

### Example 1

### (Cultivation Production of Compound A and Compound B)

A seed medium (30 mL; containing 2% corn starch, 1% glycerin, 1% sucrose, 1% Pharmamedia, 1% gluten meal and 0.2% Tween80) was poured into an Erlenmeyer flask (size: 100 mL) and sterilized by autoclaving (121 °C, 3 0 min). A loopful of the fungus Capnodiaceae strain No. 339855 was aseptically inoculated by a platinum loop from a slant culture into this type of medium, and cultured at 25°C for 4 days while shaking on a rotary shaker (220 rpm). Subsequently, a production medium (20 mL; containing 4% glucose, 6% soluble starch (Nacalai Tesque, Inc.), 2% yeast extract (Wako Pure Chemical Industries, Ltd.), 0.02% KCl, 0.02% MgSO₄·7H₂O, 1% K₂HPO₄, 1% β-cyclodextrin and 2% agar) was sterilized by autoclaving and poured into a Petri dish (diameter 9 cm). A seed culture (2 mL) was aseptically applied to the Petri dish, and cultured at 25°C for 10 days.

### (Isolation and Purification of Compound A and Compound B)

A culture (1.8 L) obtained by the above cultivation method was dipped in acetone (2 L), stirred for 1 hour, and then filtered. An equal volume of water was added to the filtrate, the resulting mixture was applied to a Diaion HP20 column (100x16 mm I.D., size: 20 mL; Mitsubishi Chemical), and the elution was carried out using a mixed solvent {acetone:water = 60:40 (v/v), 100 mL}. Water (100 mL) was added to the eluate, the resulting mixture was applied to a Daisogel SP-120-ODS-B column (size: 180 mL, 250x30 mm I.D. DAISO) and eluted with a mixed solvent {MeCN:water=50:50 (v/v), (containing 0.05% TFA)} as a mobile phase, and the resulting eluate was aliquoted into fractions including Compound A and Compound B, respectively. An equal volume of water was added to a fraction including Compound A, and the resulting mixture was applied to an OASIS HLB solid phase extraction cartridge (size: 6 g/35 cc, Waters), and eluted with MeCN (100 mL). The obtained eluate was concentrated under reduced pressure, t-BuOH was added to the concentrate, and the resulting mixture was lyophilized to obtain Compound A (265 mg) as a powder.

An equal volume of water was added to a fraction including Compound B, and the resulting mixture was applied to an OASIS HLB cartridge (size: 6 g/35 cc, Waters), and eluted with MeCN (100 mL). The obtained eluate was concentrated under reduced pressure, dissolved in a small quantity of MeOH, and purified by preparative HPLC {Column: Mightysil RP-18 GP (250×20 mm I.D.), Kanto Chemical Co., Ltd, Mobile phase; MeCN:Water=50:50 (v/v), Flow rate=10 mL/min}. A peak at the elution time of about 30 minutes was collected. The obtained eluate was concentrated under reduced pressure, t-BuOH was added to the concentrate, and the resulting mixture was lyophilized to obtain Compound B (5 mg) as a powder.

The Compound A purified and isolated by the above process showed the following physiochemical properties.

### (Physiochemical Properties of Compound A)

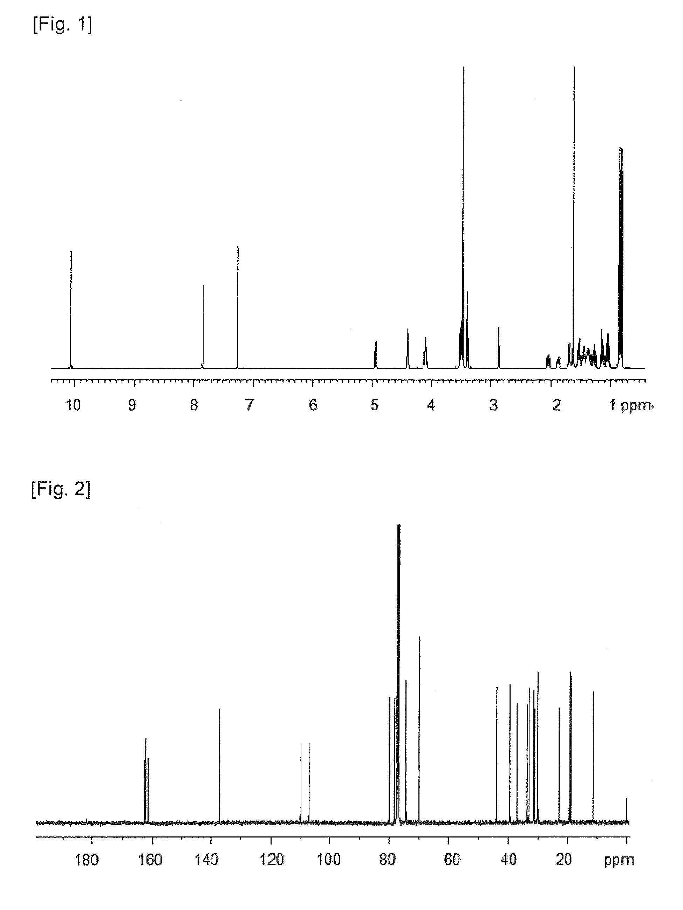

Color and Shape = White Powder
Optical rotation = [a]_{D}²⁵-120° (c 0.5, MeOH)
Molecular Formula = C₂₅H₄₁NO₇
HR ESI MS(m/z) = Calcd for C₂₅H₄₂N₁O₇ [M+H]⁺468.2961, Found 468.2965
UV = λ^{MeOH}nm(ε);233(sh), 290.5(6,100)
¹H-NMR spectrum = shown in FIG. 1.
¹³C-NMR spectrum = shown in FIG. 2.

It was concluded from the physiochemical properties of the compound A that the compound A has the following structure.

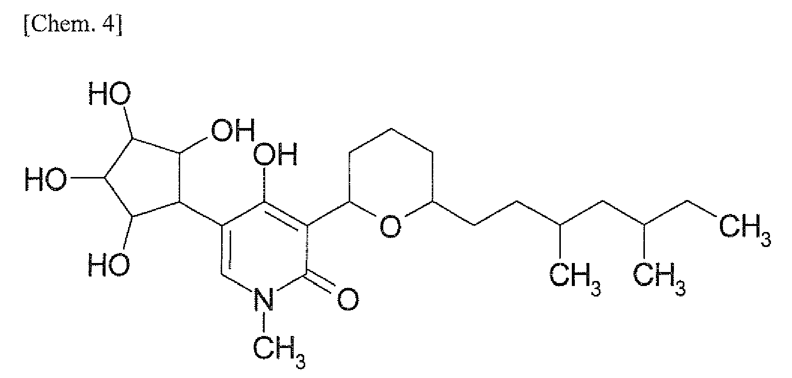

### (Physiochemical Properties of Compound B)

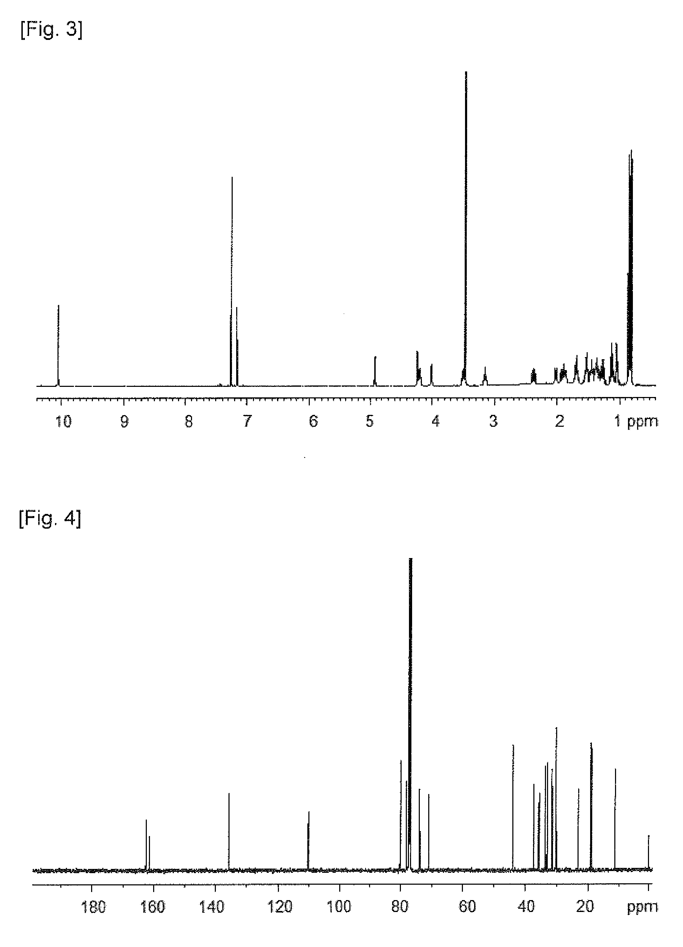

Color and Shape = White Powder
Molecular Formula = C₂₅H₄₁NO₆
HR ESI MS(m/z) = Calcd for C₂₅H₄₂N₁O₆ [M+H]⁺452.3012, Found 452.3011
¹H-NMR spectrum = shown in FIG. 3.
¹³C-NMR spectrum = shown in FIG. 4.

It was concluded from the physiochemical properties of the compound B that the compound B has the following structure.

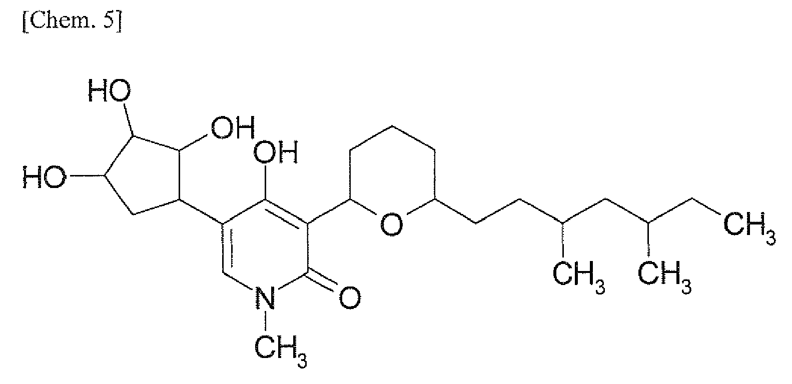

### Example 2

### (Antifungal Activity)

Antifungal activities of the compound of the present invention were confirmed by the following method.
Antifungal activities for the following test fungi were determined by a broth microdilution method (Hikaru Kume and Toshikazu Yamazaki, Clinical Microbiology, Vol. 21, No. 5, pp. 573-580, 1994). For example, the compound A showed the following activity. The minimum inhibitory concentration (MIC) of the compound A is shown in Table 1.

**[Table 1]**

| Test fungi | MIC (µg/mL) |
|---|---|
| *Trichophyton mentagrophytes* FP2103 | 0.1 |
| *Trichophyton rubrum* FP596 | 0.1 |
| *Crypto coccus neoformans* FP1739 | 1.3 |
| *Aspergillus fumigatus* FP1305 | 25 |
| *Alternaria alternata* AHU925 | 6.3 |

It was confirmed from the result of the test that the compound of the formula (I) has an antifungal activity. Therefore, it was determined that the compound is useful in treating trichophytosis, for example, trichophytic granuloma, trichophytosis unguium, tinea manuum and pedis, trichophytosis corporis, trichophytosis cruris, tinea corporis and tinea cruris, trichophytosis capitis, Celsus kerion, sycosis trichophytica, Trichophytia profunda acuta of the glabrous skin and/or acute deep-seated trichopytosis the terminal hair, etc.

### Example 3

### (Method for measuring Complex III inhibitory activity)

A medium (0.1% glucose, 0.2% bacto tryptone, 0.2% K₂HPO₄, 0,005% MgSO₄·7H₂O and 0.005% CaCl₂·2H₂O) was poured into an Erlenmeyer flask and sterilized by autoclaving. Conidiophores of *Trichophyton mentagrophytes* FP2103 were inoculated into the medium, and cultured at 30°C for 40 hours while shaking on a rotary shaker. The fungus was collected from the cultural solution and dipped in a cell wall lysis enzyme solution {1 M KCI, 25 mM CaCl₂·2H₂O, 10 mM MgCl₂·6H₂O, 5 mg/mL zymolyase-100T (Seikagaku Corporation), 1 mg/mL chitinase (Sigma) and 5 mg/mL drieselase (Sigma)}, and the resulting mixture was stirred at 30°C for 4 hours. The enzyme treatment solution was filtered through glass funnel filled with gauze to obtain protoplast. A mitochondrial fraction was isolated from the protoplast by using a Mitochondria Isolation Kit (BioChain Institute, Inc.). The Complex III inhibitory activity was measured in the ubiquinol-cytochrome c reductase assay using the isolated mitochondrial fraction (The Journal of Biological Chemistry, 279, pp. 8708-8714,2004). As a result, the compound A inhibited the complex III at 0.9 ng/mL.

It was confirmed from the result of the assay that the compound of the formula (I) or a salt thereof has a complex III inhibitory action. Therefore, it was suggested that the compound may be used for treating protozoan infection (for example, malaria, toxoplasmosis) and/or Pneumocystis pneumonia, etc.

### Example 4

### (Cytotoxicity)

Cytotoxicity was evaluated by adding a test drug to mouse T lymphoma cell line EL-4 at various concentrations, incubating the cells in a CO₂ incubator at 37°C for 72 hours, counting the cells using a cell counting kit (Wako Pure Chemical Industries, Ltd.), and calculating IC₅₀ values. As a result, for example, the compound A had an IC₅₀ value of 6.2 µg/mL and did not show a cytotoxic effect to the EL-4 cells at concentrations of 3.1 µg/mL or less.

It was suggested from the result of the assay that the compound of the formula (I) or a salt thereof has a low cytotoxic effect and fewer side effects.

### Industrial Applicability

A compound of the formula (I) or a salt thereof may be used as an agent for preventing and/or treating trichophytosis (for example, trichophytic granuloma, trichophytosis unguium, tinea manuum and pedis, trichophytosis corporis, trichophytosis cruris, tinea corporis and tinea cruris, trichophytosis capitis, Celsus kerion, sycosis trichophytica, Trichophytia profunda acuta of the glabrous skin and/or acute deep-seated trichopytosis the terminal hair, etc.), protozoan infection (for example, malaria, toxoplasmosis, etc.) and/or Pneumocystis pneumonia (formerly: carinii pneumonia), etc.

## Claims

1. A compound of the formula (I) or a salt thereof 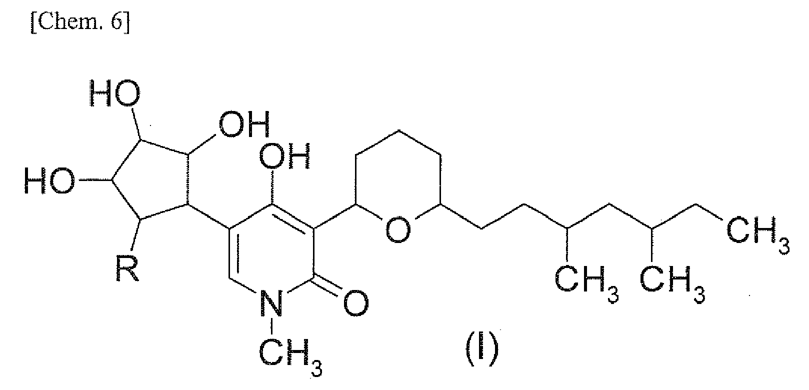 (wherein, R represents H or OH).

2. The compound or the salt thereof of claim 1, wherein R is OH.

3. The compound or the salt thereof of claim 1, wherein R is H.

4. A pharmaceutical composition comprising the compound as described in claim 1 or the salt thereof, and a pharmaceutically acceptable excipient.

5. The pharmaceutical composition of claim 4, which is an agent for preventing or treating trichophytosis, protozoan infection and/or Pneumocystis pneumonia.

6. The composition or the salt thereof of claim 1, which is for use in preventing or treating trichophytosis, protozoan infection and/or Pneumocystis pneumonia.

7. A method for preventing or treating trichophytosis, protozoan infection and/or Pneumocystis pneumonia, which comprises administering an effective amount of the compound as described in claim 1 or the salt thereof to a patient.

8. Capnodiaceae strain No. 339855 deposited as an Accession No. NITE BP-735 or a variant thereof.

9. A method for preparing the compound as described in claim 1 or a salt thereof, which comprises culturing a microorganism that belongs to the family Capnodiaceae capable of producing the compound as described in claim 1, and isolating the compound as described in claim 1 or the salt thereof from a culture thereof.

10. The method of claim 9, wherein the microorganism is a microorganism as described in claim 8.
